# EUROPÄISCHE PATENTANMELDUNG

(11) **EP 4 397 293 A1**
(43) Veröffentlichungstag der Anmeldung: **10.07.2024**
(21) Anmeldenummer: 23152276.4
(22) Anmeldetag: 18.01.2023
(51) Int. Cl.: A61H 33/06, A61N 5/06

(54) **INFRAROTSTRAHLER**

(30) Priorität: 09.01.2023 EP 23150709
(71) Anmelder: Mattheiss, Gerd, 7203 Trimmis (CH)
(72) Erfinder: PECHER, Otto, 85653 Aying b. München (DE); ZEIGER, Thomas, 6134 Vomp (AT); MATTHEISS, Gerd, 7203 Trimmis (CH); PIPPIG, Marcus, 08541 Theuma (DE)
(74) Vertreter: Fuchs Patentanwälte Partnerschaft mbB

(57) **Zusammenfassung**

Eine Benutzeraufnahme (300) für ein Wärmebehandlungssystem (400) ist so ausgebildet, dass eine Infrarotstrahler-Anordnung (100) des Wärmebehandlungssystems (400) an der Benutzeraufnahme (300) anbringbar ist. Die Benutzeraufnahme (300) umfasst dabei wenigstens eine Anliegefläche (302), die dem Benutzer zugewandt ist und die eine Aussparung (304) aufweist, die mit einem zur Wärmebehandlung vorgesehenen Hautbereich des Benutzers in einer Bestrahlungsrichtung wenigstens teilweise überlappt. Die Infrarotstrahler-Anordnung (100) ist in einem von der Anliegefläche (302) abgewandten Bereich (306) der Benutzeraufnahme (300) anordenbar derart, dass Infrarotstrahlung, die mittels der wenigstens einen Wärmequelle (150, 152) erzeugt ist, durch die Aussparung (304) in Richtung auf den Benutzer tritt.

## Beschreibung

Die Anmeldung betrifft das Gebiet der Wärmebehandlungssysteme. Die Anmeldung betrifft insbesondere eine Benutzeraufnahme für ein Wärmebehandlungssystem, ein Wärmebehandlungssystem und eine Wärmekabine umfassend ein Wärmebehandlungssystem.

### Technischer Hintergrund

Wärmebehandlungssysteme sind bekannt. Sie werden üblicherweise dazu verwendet, Wärme in Form von Wärme- bzw. Infrarotstrahlung auf den Körper oder bestimmte Körperregionen eines Benutzers aufzubringen. Eine Wärmebehandlung geschieht oft als therapeutische oder therapiebegleitende Maßnahme, etwa im Rahmen physiotherapeutischer Behandlungen, oder allgemein zur Förderung des Wohlbefindens oder der Erholung.

Es besteht ein fortwährender Bedarf nach verbesserten Techniken zur Wärmebehandlung.

### Abriss der Erfindung

Diese Aufgabe wird durch eine Benutzeraufnahme für ein Wärmebehandlungssystem gemäß Anspruch 1, ein Wärmebehandlungssystem gemäß Anspruch 12 und eine Wärmekabine gemäß Anspruch 15 gelöst.

Demnach wird gemäß einem ersten Aspekt eine Benutzeraufnahme für ein Wärmebehandlungssystem vorgestellt. Die Benutzeraufnahme ist so ausgebildet, dass eine Infrarotstrahler-Anordnung des Wärmebehandlungssystems an der Benutzeraufnahme anbringbar ist. Die Benutzeraufnahme umfasst wenigstens eine Anliegefläche, die dem Benutzer zugewandt ist und die eine in ihrer Größe und/oder Form verstellbare Aussparung aufweist, die mit einem zur Wärmebehandlung vorgesehenen Hautbereich des Benutzers in einer Bestrahlungsrichtung wenigstens teilweise überlappt. Die Infrarotstrahler-Anordnung ist in einem von der Anliegefläche abgewandten Bereich der Benutzeraufnahme anordenbar derart, dass Infrarotstrahlung, die mittels der wenigstens einen Wärmequelle erzeugt ist, durch die in ihrer Größe und/oder Form verstellbare Aussparung in Richtung auf den Benutzer tritt. In ihrer Größe und/oder Form verstellbar kann sich insbesondere auf die Fläche der Aussparung beziehen.

Eine solche Benutzeraufnahme ermöglicht eine verbesserte Anpassung des Wärmebehandlungssystems an ein jeweiliges Behandlungserfordernis. Das betrifft eine mögliche Anpassung der Benutzeraufnahme an einen jeweiligen Benutzer und/oder an einen vorgesehenen Bestrahlungsbereich. Es betrifft außerdem eine mögliche Anpassung, insbesondere Auswahl, einer Infrarotstrahler-Anordnung zum Anbringen an die Benutzeraufnahme. Eine solche Benutzeraufnahme ermöglicht außerdem eine vereinfachte Wartung und eine vereinfachte Reinigung der Infrarotstrahler-Anordnung. Die Benutzeraufnahme kann ferner wenigstens ein Blendenelement umfassen, dessen Position in Bezug auf die Infrarotstrahler-Anordnung verstellbar ist, um einen Bestrahlungsbereich der Infrarotstrahler-Anordnung verstellbar zu begrenzen. Hierdurch kann die Größe und/oder Form der Aussparung variabel verstellt bzw. angepasst werden.

Die Benutzerauflage kann wenigstens ein verstellbares Anliegeelement umfassen, das die Aussparung, insbesondere zumindest einen Bereich der Aussparung, in wenigstens einer Richtung verstellbar begrenzt. Hierdurch kann die Größe und/oder Form der Aussparung variabel verstellt bzw. angepasst werden.

Das wenigstens eine verstellbare Anliegeelement kann gemäß einer Körpergröße des Benutzers und/oder gemäß einer Körperregion des zur Wärmebehandlung vorgesehenen Hautbereichs des Benutzers verstellbar sein. Entsprechend kann die Größe und/oder Form der Aussparung gemäß einer Körpergröße des Benutzers und/oder gemäß einer Körperregion des zur Wärmebehandlung vorgesehenen Hautbereichs des Benutzers verstellbar sein.

Durch Verstellen der Größe und/oder der Form der Aussparung gemäß einer Körpergröße des Benutzers lässt sich ein Verletzungsrisiko für den Benutzer verringern. Ein Verletzungsrisiko, das sich so verringern lässt, kann ein Verletzungsrisiko umfassen, das sich bei Benutzern von verhältnismäßig geringer Körpergröße, insbesondere bei Kindern oder jungen Jugendlichen, infolge eines möglichen Durchrutschens von Körperteilen des Benutzers durch die Aussparung auf erhitzte Bereiche der Infrarotstrahler-Anordnung ergibt. Eine Verwendung der Benutzeraufnahme für ein Wärmebehandlungssystem durch Benutzer mit geringer Körpergröße lässt sich so ermöglichen oder zumindest begünstigen.

Das wenigstens eine Blendenelement kann an einer von der Anliegefläche abgewandten Seite des wenigstens einen verstellbaren Anliegeelements angeordnet sein. Die Position des Blendenelements in Bezug auf die Infrarotstrahler-Anordnung kann dabei durch Verstellen des verstellbaren Anliegeelements verstellbar sein.

Das wenigstens eine verstellbare Anliegeelement mit dem wenigstens einen Blendenelement kann verstellbar sein, um den Bestrahlungsbereich der Infrarotstrahler-Anordnung gemäß einer Größe des zur Wärmebehandlung vorgesehenen Hautbereichs des Benutzers verstellbar zu begrenzen. Entsprechend kann die Größe und/oder Form der Aussparung gemäß einer Körpergröße des Benutzers und/oder gemäß einer Körperregion des zur Wärmebehandlung vorgesehenen Hautbereichs des Benutzers verstellbar sein.

Die Benutzeraufnahme kann eine Stützstruktur in Form eines Sitzes, einer Liege und/oder einer Lehne umfassen und zur sitzenden, liegenden und/oder stehenden Aufnahme des Benutzers vorgesehen sein.

Die Anliegefläche kann einer dem Benutzer zugewandten Seite der Stützstruktur entsprechen. Die Infrarotstrahler-Anordnung kann dabei im Bereich einer von der Anliegefläche abgewandten Seite der Stützstruktur anbringbar sein.

Die Aussparung kann sich in einem Bereich der Stützstruktur erstrecken, der einem Rückenbereich des Benutzers entspricht.

Das wenigstens eine Blendenelement und/oder das wenigstens eine verstellbare Anliegeelement kann in wenigstens einer aus einer vertikalen und einer lateralen Richtung verstellbar sein. Das kann vorgesehen sein, um eine Größe des zur Bestrahlung vorgesehenen Hautbereichs im Rückenbereich des Benutzers einzustellen und/oder um die Stützstruktur einer Körpergröße des Benutzers anzupassen.

Die Benutzeraufnahme kann wenigstens zwei Blendenelemente und/oder wenigstens zwei verstellbare Anliegeelemente umfassen, die lateral zu verschiedenen Seiten eines Mittenbereichs der Aussparung angeordnet sind.

Gemäß einem weiteren Aspekt wird ein Wärmebehandlungssystem vorgestellt. Das Wärmebehandlungssystem umfasst eine Benutzeraufnahme der hier vorgestellten Art, und eine Infrarotstrahler-Anordnung, die in Bezug auf die Anliegefläche der Benutzeraufnahme derart angeordnet ist, dass Infrarotstrahlung, die mittels der Infrarotstrahler-Anordnung erzeugt ist, durch die Aussparung in Richtung auf den Benutzer tritt.

Die Infrarotstrahler-Anordnung des Wärmebehandlungssystems kann wenigstens eine Wärmequelle, wenigstens einen Reflektor, der dazu ausgebildet ist, Infrarotstrahlung, die mittels der wenigstens einen Wärmequelle erzeugt ist, in Richtung auf den Benutzer der Wärmebehandlungssystems umzulenken, und wenigstens ein Gestell, an dem die wenigstens eine Wärmequelle und der wenigstens eine Reflektor angebracht sind, umfassen.

Während einer vorgesehenen Verwendung des Wärmebehandlungssystems kann dabei der wenigstens eine Reflektor mechanischer und/oder chemischer Wechselwirkung mit einer Umgebung des Wärmebehandlungssystems ausgesetzt sein. Zudem kann der wenigstens eine Reflektor reversibel lösbar an dem wenigstens einen Gestell angebracht sein.

Die wenigstens eine Wärmequelle kann eine steuerbare Wärmequelle sein.

Das Wärmebehandlungssystem kann dabei ferner wenigstens einen Wärmesensor, der in der Infrarotstrahler-Anordnung aufgenommen ist und der dazu ausgebildet ist, während der vorgesehenen Verwendung des Wärmebehandlungssystems eine Temperatur in dem Hautbereich des Benutzers zu erfassen und ein Sensorsignal auszugeben, das auf die erfasste Temperatur hinweist, und eine Steuerungseinrichtung, die dazu ausgebildet ist, das Sensorsignal zu empfangen und wenigstens teilweise auf der Grundlage des empfangenen Sensorsignals die wenigstens eine Wärmequelle in Bezug auf eine Heizleistung zu steuern, umfassen.

Die wenigstens eine Wärmequelle, der wenigstens eine Reflektor, der wenigstens eine Wärmesensor und die Steuerungseinrichtung können dabei Bestandteile einer Wärmebehandlungsvorrichtung des Wärmebehandlungssystems sein.

Gemäß einem weiteren Aspekt wird eine Wärmekabine vorgestellt. Die Wärmekabine umfasst ein Wärmebehandlungssystem der hier vorgestellten Art.

### Kurze Beschreibung der Zeichnungen

Weitere Aufgaben, Merkmale und Vorzüge der Erfindung werden anhand der Zeichnungen und der ausführlichen Beschreibung deutlich. Es zeigen:
- Fig. 1: eine Schrägansicht einer Infrarotstrahler-Anordnung gemäß einem Beispiel;
- Fig. 2: eine Explosionsansicht der Infrarotstrahler-Anordnung aus Fig. 1;
- Fig. 3A und 3B: Vorderansichten verschiedener Konfigurationen einer Benutzeraufnahme für ein Wärmebehandlungssystem gemäß einem Beispiel;
- Fig. 4: eine Seitenansicht eines Wärmebehandlungssystems gemäß einem Beispiel;
- Fig. 5: eine Wärmekabine gemäß einigen Beispielen, und
- Fig. 6: eine Wärmebehandlungsvorrichtung gemäß einem Beispiel.

### Ausführliche Beschreibung

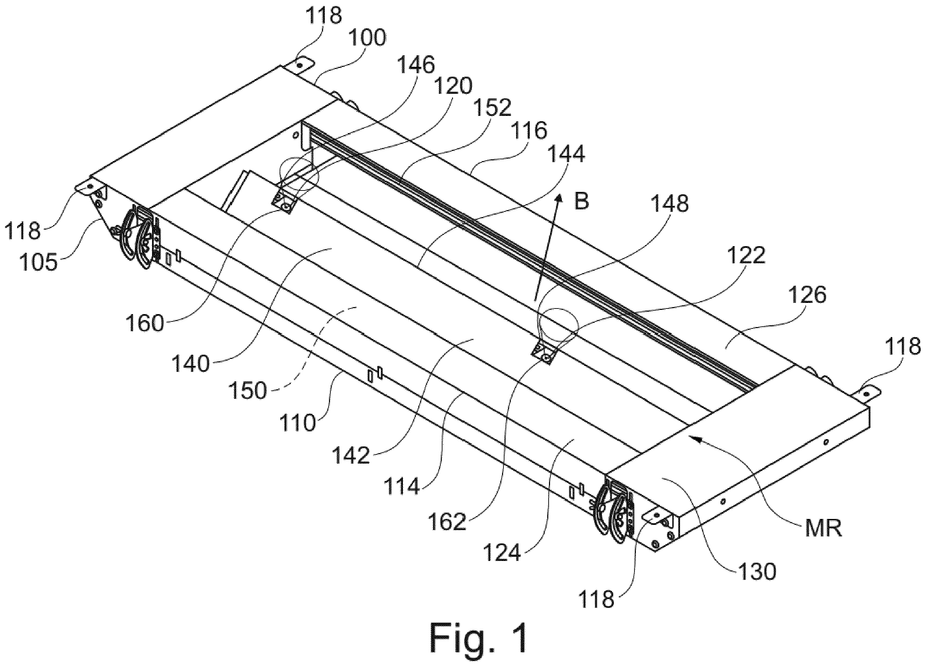

Fig. 1 zeigt schematisch und exemplarisch eine Schrägansicht einer Infrarotstrahler-Anordnung 100. Die Infrarotstrahler-Anordnung 100 ist zur Verwendung in einem Wärmebehandlungssystem und/oder einer Wärmebehandlungsvorrichtung, wie im Nachfolgenden jeweils beschrieben, vorgesehen.

Die Infrarotstrahler-Anordnung 100 umfasst ein Gestell 105, in dem mehrere Wärmequellen 150, 152 und ein Reflektor 140 angeordnet sind. Die Wärmequellen 150, 152 sind in Seitengehäusen 114, 116 des Gestells 105 untergebracht. Der Reflektor 140 ist dabei zwischen den Wärmequellen 150, 152 angeordnet. Der Reflektor 140 ist dazu vorgesehen, Infrarotstrahlung, die mittels der Wärmequellen 150,152 erzeugt wird und die so aus verschiedenen seitlichen Richtungen auf den Reflektor 140 trifft, in eine Bestrahlungsrichtung B der Infrarotstrahler-Anordnung 100 umzulenken. Um ein Austreten der umgelenkten Infrarotstrahlung aus der Infrarotstrahler-Anordnung 100 zu gestatten, weist das Gestell 105 ausgehend von dem Reflektor 140 in der Bestrahlungsrichtung B eine Öffnung 112 auf.

In dem gezeigten Beispiel erstreckt sich die Öffnung 112 zwischen den Oberseiten der Seitengehäuse 114, 116 sowie im Wesentlichen über eine gesamte Erstreckungsfläche des Reflektors 140. Der Reflektor 140 ist zudem reversibel lösbar an dem Gestell 105 angebracht, beispielsweise durch elastisches Klemmen oder Verschrauben. Die Öffnung 112 ermöglicht in dem gezeigten Beispiel ein Entnehmen des Reflektors 140 aus dem Gestell 105 sowie ein Einsetzen des Reflektors 140 in das Gestell 105 jeweils durch die Öffnung 112. Dadurch ist beispielsweise ein Austauschen und/oder Reinigen des Reflektors 140 begünstigt. In anderen Beispielen erfolgen das Entnehmen und das Einsetzen des Reflektors 140 auf anderem Weg als durch die Öffnung 112, beispielsweise durch eine Öffnung an einer Seite des Gestells 105 und/oder mittels eines reversibel entfernbaren Bodens 110 des Gestells 105.

Die Infrarotstrahler-Anordnung 100 weist in einem Randbereich des Gestells 105 mehrere Befestigungsmittel 118 auf. Die Befestigungsmittel 118 dienen dazu, die Infrarotstrahler-Anordnung 100 in einem Wärmebehandlungssystem, beispielsweise an einer Benutzeraufnahme eines Wärmebehandlungssystems, wie im Nachfolgenden beschrieben, zu befestigen.

Mittels der beschriebenen Anordnung von Wärmequellen 150, 152 und Reflektor 140 ermöglicht die Infrarotstrahler-Anordnung 100 das Aufbringen von Wärme in Form von Infrarotstrahlung auf einen Benutzer, bzw. einen Hautbereich des Benutzers, der sich in einem Bestrahlungsbereich der Infrarotstrahler-Anordnung 100 in der Bestrahlungsrichtung B befindet.

Die Anordnung von Wärmequellen 150, 152 und Reflektor 140 begünstigt dabei eine effiziente Bestrahlung unter vielfältigen Einsatzbedingungen der Infrarotstrahler-Anordnung 100 bei gleichzeitig verbesserter Sauberkeit und Hygiene. Das ist teilweise dadurch erzielt, dass eine transparente Schutzabdeckung der Öffnung 112, etwa in Form eines Deckglases, wie sie bei herkömmlichen Infrarotstrahler-Anordnungen typischerweise vorgesehen sind, vermeidbar ist. Die mittels der Wärmequellen 150, 152 erzeugte Infrarotstrahlung wird demnach auch nicht durch eine solche Schutzabdeckung teilweise absorbiert.

In Hinsicht auf eine Benutzungssicherheit ist das Vermeiden einer Schutzabdeckung dadurch ermöglicht, dass die Wärmequellen 150, 152 vor versehentlicher Berührung geschützt, beispielsweise in den Seitengehäusen 114, 116, untergebracht werden können.

In Hinsicht auf Sauberkeit und Hygiene ist eine Schutzabdeckung bei der Infrarotstrahler-Anordnung 100 dadurch vermeidbar, dass Bereiche der Infrarotstrahler-Anordnung 100, die bei einer Benutzung besonderer mechanischer und/oder chemischer Wechselwirkung mit der Umgebung, beispielweise Schweißabsonderungen des Benutzers, ausgesetzt sind, nämlich der Reflektor 140, für eine Reinigung leicht zugänglich und reversibel aus dem Gestell 105 entfernbar sind.

Die beschriebene Anordnung begünstigt so besonders einen Einsatz der Infrarotstrahler-Anordnung 100 mit aufwärts gerichteter und/oder aufwärts geneigter Bestrahlungsrichtung B. Das ist beispielsweise vorteilhaft, wenn die Infrarotstrahler-Anordnung 100 für Wärmebehandlungen im Rückenbereich eines Benutzers vorgesehen ist und zu diesem Zweck in einer geneigten und/oder neigbaren Rückenlehne einer zur sitzenden und/oder liegenden Aufnahme des Benutzers ausgebildeten Benutzeraufnahme angeordnet ist. Unter solchen und ähnlichen Einsatzbedingungen ist der Reflektor 140 üblicherweise in erhöhtem Maß Schweißabsonderungen des Benutzers ausgesetzt.

An der dem Benutzer zugewandten Seite jedes der Seitengehäuse 114, 116 ist jeweils eine Schweißabtropfkontur 124, 126 angeordnet. Die Schweißabtropfkonturen 124, 126 sind dazu vorgesehen, Schweißabsonderungen des Benutzers, die außerhalb der Öffnung 112 auf die Infrarotstrahler-Anordnung 100 treffen oder sich dort niederschlagen, durch die Öffnung 112 in Richtung auf den Reflektor 140 zu leiten. Eine Verunreinigung der Infrarotstrahler-Anordnung 100 an der Außenseite sowie einer Umgebung der Infrarotstrahler-Anordnung 100 infolge von Schweißabsonderungen lässt sich so verringern. Zudem ist dadurch ein Auffangen von Schweiß des Benutzers, beispielsweise zum Zweck einer Analyse, wie nachfolgend beschrieben, begünstigt.

In einem Bereich des Gestells 105, der bei einer vorgesehenen Montage der Infrarotstrahler-Anordnung 100 einem unteren Bereich des Gestells 105 entspricht, umfasst das Gestell 105 einen Schweißauffang 130. Der Schweißauffang 130 ist dazu vorgesehen, während einer Wärmebehandlung Schweißabsonderungen des Benutzers, die über die Schweißabtropfkonturen 124, 126 sowie direkt durch die Öffnung 112 auf den Reflektor 140 treffen und von dort ablaufen, aufzufangen. Der so aufgefangene Schweiß lässt sich hygienisch entsorgen. Nach Bedarf lässt sich der so aufgefangene Schweiß zudem chemisch analysieren, etwa um Rückschlüsse auf einen körperlichen Zustand des Benutzers zu gestatten.

Die beschriebene Anordnung von Reflektor 140 und in Bezug auf die Bestrahlungsrichtung B jeweils seitlich davon untergebrachten Wärmequellen 150, 152 ermöglicht darüber hinaus die Verwendung von hauptsächlich umgelenkter anstatt direkter Infrarotstrahlung für eine Wärmebehandlung. Eine gleichmäßige bzw. auf eine vorgesehene Verwendung abgestimmte Wärmeverteilung über den Bestrahlungsbereich ist so begünstigt.

In dem gezeigten Beispiel ermöglicht die Anordnung der Wärmequellen 150, 152 in den Seitengehäusen 114, 116 zudem eine Abschattung des Bestrahlungsbereichs vor direkter Infrarotstrahlung der Wärmequellen 150, 152 mittels der Seitengehäuse 114, 116. Der Anteil umgelenkter anstatt direkter Infrarotstrahlung für eine Wärmebehandlung lässt sich so weiter erhöhen bzw. durch geeignete Wahl der Seitengehäuse 114, 116 einstellen.

Die Wärmequellen 150, 152 sind zu verschiedenen Seiten des Reflektors 140 untergebracht. Um dabei die Infrarotstrahlung jeder der Wärmequellen 150, 152 in die Bestrahlungsrichtung B umzulenken, weist der Reflektor mehrere Reflektionsflächen 142, 144 auf, die verschieden ausgerichtet sind. Die Reflektionsflächen 142, 144 erstrecken sich jeweils angrenzend an einen Mittenbereich MR des Reflektors 140. Die Reflektionsflächen 142, 144 sind so angeordnet, dass wenigstens ein Teil der Infrarotstrahlung, die von jedweder der Wärmequellen 150, 152 abgestrahlt wird, auf jeweils wenigstens eine der Reflektionsflächen 142, 144, die der jeweiligen Wärmequelle 150, 152 zugewandt angeordnet ist, trifft und in die Bestrahlungsrichtung B umgelenkt wird. Dabei sind die Reflektionsflächen 142, 144 beispielsweise dachförmig bzw. in Form eines umgedrehten ,V' angeordnet. Infrarotstrahlung, die von den Wärmequellen 150, 152 seitlich auf jeweils eine der Reflektionsflächen 142, 144 trifft, wird so mittels der Reflektionsflächen 142, 144 in die Bestrahlungsrichtung B umgelenkt.

Die Anordnung der Wärmequellen 150, 152 zu verschiedenen Seiten des Reflektors 140 begünstigt einen breiten Bestrahlungsbereich bei zugleich kompakter, insbesondere flacher, Ausgestaltung der Infrarotstrahler-Anordnung 100. Die Anordnung der Wärmequellen 150, 152 zu verschiedenen Seiten des Reflektors 140 begünstigt dabei außerdem eine gleichmäßige, insbesondere symmetrische, Wärmeverteilung über die Breite des Bestrahlungsbereichs. Das ist vorteilhaft, beispielsweise bei typischen Wärmebehandlungen im Bereich der Wirbelsäule.

Die im Beispiel von Fig. 1 gezeigte längliche Erstreckung der Wärmequellen 150, 152, des Reflektors 140 und der Öffnung 112 begünstigt außerdem einen entsprechend langgestreckten Behandlungsbereich mit gleichmäßiger Wärmeverteilung über die Länge des Behandlungsbereichs. Das ist vorteilhaft, beispielsweise für Wärmebehandlungen entlang einem längeren Wirbelsäulenabschnitt.

Die Infrarotstrahler-Anordnung 100 begünstigt so zudem eine verhältnismäßig gleichmäßige Leistungsdichte der Infrarotstrahlung in einem Volumen oberhalb eines zu behandelnden Hautbereichs, beispielsweise einem Volumen, das sich angrenzend an den Hautbereich von einer tiefsten Stelle in Strahlungsrichtung gemessen um etwa 10 cm entgegen der Bestrahlungsrichtung erstreckt. Selbst bei Unebenheiten des bestrahlten Hautbereichs, wie sie beispielsweise durch die Wirbelsäulenkrümmungen auftreten, ist so ein gleichmäßiger Wärmeeintrag in dem gesamten Hautbereich möglich.

In dem Beispiel von Fig. 1 umfasst die Infrarotstrahler-Anordnung 100 zudem Wärmesensoren 160, 162. Diese sind entlang dem Mittenbereich MR des Reflektors 140 zwischen den Reflektionsflächen 142, 144 angeordnet. Der Reflektor 140 weist dazu Reflektoraussparungen 146, 148 auf, unter denen jeweils auf einem Boden 110 des Gestells 105 eine Wärmesensoraufnahme 120, 122 angeordnet ist. Das gestattet eine Anordnung der Wärmesensoren 160, 162 hinter den Reflektionsflächen 142, 144. Auf diese Weise sind die Wärmesensoren 160, 162 vor direkter Infrarotstrahlung, die von den Wärmequellen 150, 152 abgestrahlt wird und die sonst ein Messergebnis der Wärmesensoren 160, 162 verfälschen könnte, abgeschirmt. Die Wärmesensoren 160, 162 sind bevorzugt so ausgerichtet, dass ein Messbereich jedes der Wärmesensoren 160, 162 mit dem Bestrahlungsbereich der Infrarotstrahler-Anordnung 100 wenigstens teilweise überlappt.

Mittels der Wärmesensoren 160, 162 lässt sich eine Oberflächentemperatur des Hautbereichs während des Aufbringens von Infrarotstrahlung in dem Hautbereich erfassen. Eine Wirkung der Infrarotstrahlung auf die Oberflächentemperatur in dem bestrahlten Hautbereich lässt sich so, beispielsweise mithilfe einer Steuerungseinrichtung, wie nachfolgend beschrieben, etwa kontinuierlich oder in beliebigen zeitlichen Abständen, sensorisch überwachen.

In einigen Beispielen der Infrarotstrahler-Anordnung 100 sind die Wärmequellen 150, 152 als steuerbare Wärmequellen, beispielsweise steuerbare Halogenröhren, ausgebildet. In Verbindung mit einer sensorischen Erfassung der Oberflächentemperatur in dem behandelten Hautbereich lässt sich die Heizleistung der Wärmequellen 150, 152, beispielsweise mithilfe einer Steuerungseinrichtung, in einigen Beispielen automatisch regeln.

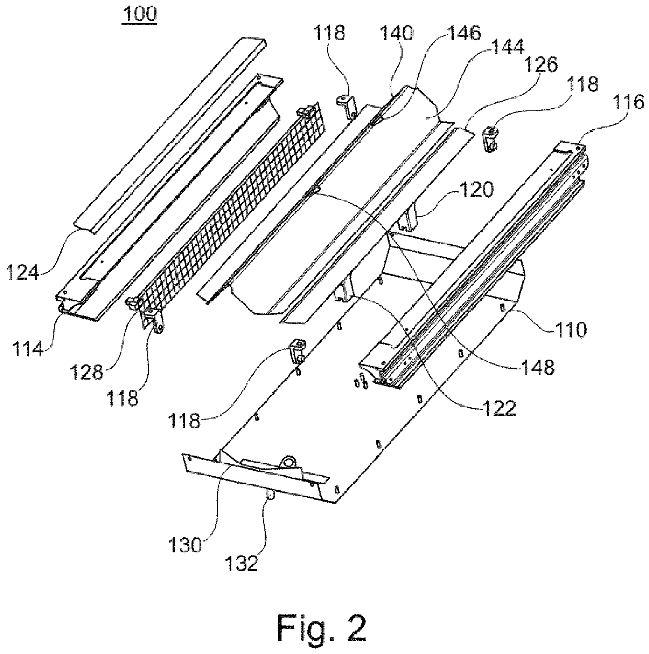

Fig. 2 zeigt schematisch eine Explosionsansicht der Infrarotstrahler-Anordnung 100. Gleiche Bezugszeichen wie in Fig. 1 bezeichnen dabei gleiche Merkmale.

Wie in Fig. 2 erkennbar, ist der Schweißauffang 130 schalenförmig im unteren Bereich des Bodens 110 ausgebildet. Über einen Schweißauslass 132 lässt sich aufgefangener Schweiß aus dem Schweißauffang 130 abführen.

Fig. 2 zeigt ferner exemplarisch ein Schutzgitter 128. Ein solches Schutzgitter 128 kann je nach Erfordernis in einigen Beispielen vor jeder der Wärmequellen 150, 152 zum besseren Schutz vor unbeabsichtigter Berührung der Wärmequellen 150, 152 durch einen unachtsamen Benutzer vorgesehen sein.

Die Schweißabtropfkonturen 124, 126 sind jeweils als separate Teile ausgebildet, die an den Seitengehäusen 114, 116 angebracht sind. Die Schweißabtropfkonturen 124, 126 dienen dabei in einigen Beispielen zugleich als Federelemente, die bei einem Anbringen der Infrarotstrahler-Anordnung 100 an einer Benutzeraufnahme das Gehäuse 105 gegenüber der Benutzeraufnahme federnd abstützen.

Erkennbar ist in Fig. 2 zudem der als separates Teil, beispielsweise als Reflektorblech und/oder aus Metall, sowie reversibel vom Gehäuse 105 lösbar ausgebildete Reflektor 140.

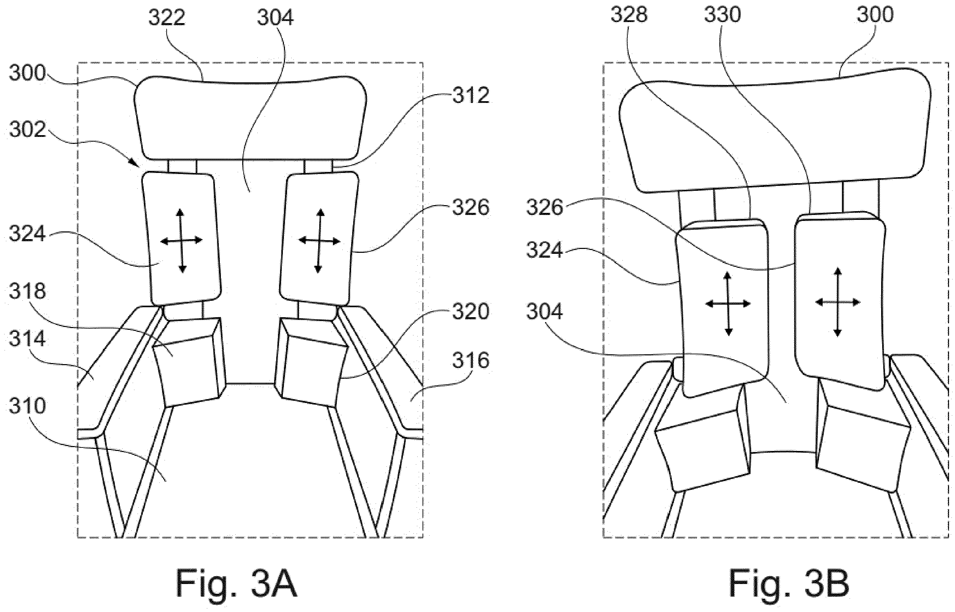

Fig. 3A und 3B zeigen schematisch und exemplarisch eine Benutzeraufnahme 300. Die Benutzeraufnahme 300 ist zur Verwendung in einem Wärmebehandlungssystem vorgesehen, beispielsweise in Verbindung mit der Infrarotstrahler-Anordnung 100 wie voranstehend im Zusammenhang mit Fig. 1 und 2 beschrieben. In dem gezeigten Beispiel ist die Benutzeraufnahme 300 zur sitzenden Aufnahme eines Benutzers vorgesehen. Die Benutzeraufnahme 300 umfasst zu diesem Zweck eine Stützstruktur in Form eines Sitzes.

Die Benutzeraufnahme 300 weist eine dem Benutzer zugewandte Anliegefläche 302 auf. Darin ist eine Aussparung 304 gemäß einer zur Wärmebehandlung vorgesehenen Körperregion des Benutzers vorgesehen. Die Benutzeraufnahme 300 umfasst zudem eine Sitzfläche 310, eine Rückenlehne 312 und Armlehnen 314, 316. Die Aussparung 304 ist im Bereich der Rückenlehne 312 angeordnet. Die Aussparung 304 ist dabei seitlich und nach oben durch Lendenstützen 318, 320, eine Nackenstütze 322 sowie durch der Höhe nach dazwischen angeordnete verstellbare Anliegeelemente 324, 326 begrenzt.

Die Benutzeraufnahme 300 ist dazu vorgesehen, dass eine Infrarotstrahler-Anordnung, beispielsweise die Infrarotstrahler-Anordnung 100, in einem von der Anliegefläche 302 abgewandten Bereich der Benutzeraufnahme 300 angebracht wird. Eine Bestrahlung mit Infrarotstrahlung mittels der Infrarotstrahler-Anordnung erfolgt dabei durch die Aussparung 304.

Die verstellbaren Anliegeelemente 324, 326 gestatten dabei, die Anliegefläche 302 an die Körperform, beispielsweise die Körpergröße, des jeweiligen Benutzers anzupassen. Dadurch ist eine geeignete Stützung des Körpers des Benutzers während einer Wärmebehandlung erzielbar. In dem gezeigten Beispiel lässt sich so beispielsweise mittels der verstellbaren Anliegelemente 324, 326 eine geeignete Stützung der Kyphose des Benutzers erzielen.

Ein Anpassen der Anliegefläche 302 an die Körperform, insbesondere die Körpergröße, mittels der verstellbaren Anliegeelemente 324, 326 gestattet außerdem, ein Verletzungsrisiko für den Benutzer zu verringern. Ein solches Verletzungsrisiko kann sich insbesondere bei Benutzern von verhältnismäßig geringer Körpergröße, beispielsweise bei Kindern oder jungen Jugendlichen, infolge eines möglichen Durchrutschens von Körperteilen des Benutzers durch die Aussparung 304 auf erhitzte Bereiche der Infrarotstrahler-Anordnung 100 ergeben.

Die verstellbaren Anliegeelemente 324, 326 gestatten in dem gezeigten Beispiel außerdem, den Bestrahlungsbereich an ein jeweiliges Erfordernis, beispielsweise gemäß der Körpergröße und/oder eines zur Behandlung vorgesehenen Körperbereichs, anzupassen. Dabei findet eine Begrenzung des Bestrahlungsbereichs mittels der verstellbaren Anliegeelemente 324, 326 durch Abschatten von nicht zur Behandlung vorgesehenen Hautpartien statt. Zu diesem Zweck weisen die verstellbaren Anliegeelemente 324, 326 an ihrer Rückseite, d.h. an der der Anliegefläche 302 abgewandten Seite, Blendenelemente 328, 330, beispielsweise aus Metall, auf.

In anderen Beispielen von Benutzeraufnahmen der vorgestellten Art sind zusätzlich oder alternativ verstellbare Blendenelemente getrennt von verstellbaren Anliegeelementen ausgebildet. Dadurch ist ermöglicht, ein Anpassen des Bestrahlungsbereichs mittels der verstellbaren Blendenelemente wenigstens teilweise unabhängig von einem Anpassen der Anliegefläche an die Körperform des Benutzers, das mittels der verstellbaren Anliegeelemente erfolgen kann, vorzunehmen.

Um ein Verstellen der Anliegeelemente 324, 326 und/oder der Blendenelemente 328, 330 zu ermöglichen, sind diese in einigen Beispielen jeweils mittels wenigstens einer lösbaren Verbindung an der Stützstruktur der Benutzeraufnahme 300 angebracht. Eine lösbare Verbindung umfasst dabei beispielsweise eine Schraubverbindung, eine Bolzenverbindung, eine Klemmverbindungen, eine Magnetverbindung und/oder eine Bajonettverbindung.

Das Verstellen der Anliegeelemente 324, 326 und der Blendenelemente 328, 330 erfolgt in dem gezeigten Beispiel durch Verändern einer Position der jeweils wenigstens einen lösbaren Verbindung. In anderen Beispielen erfolgt das Verstellen der Anliegeelemente und/oder der Blendenelemente zusätzlich oder alternativ durch geeignetes Auswählen der Anliegeelemente und/oder der Blendenelemente aus einer Vielzahl verschieden ausgebildeter Anliegeelemente und/oder Blendenelemente sowie durch lösbares Verbinden der ausgewählten Anliegeelemente und/oder Blendenelemente mit der Stützstruktur der Benutzeraufnahme 300.

Wie in Fig. 3A und 3B durch die gekreuzten Doppelpfeile angedeutet, ist jedes der verstellbaren Anliegeelemente 324, 326 sowohl in vertikaler Richtung als auch in lateraler Richtung verstellbar. Fig. 3A zeigt dabei die verstellbaren Anliegeelemente 324, 326 auf einer mittleren Höhe bei breitestmöglicher Anliegefläche 302 im Bereich der Kyphose. Das entspricht zugleich einem breitestmöglichen Bestrahlungsbereich durch die Aussparung 304.

Fig. 3B zeigt dagegen die verstellbaren Anliegeelemente 324, 326 ebenfalls auf einer mittleren Höhe, aber bei schmalster Anliegefläche 302. Das entspricht zugleich einem schmalsten Bestrahlungsbereich durch die Aussparung 304.

Das in Fig. 3A und 3B gezeigte Beispiel der Benutzeraufnahme 300 ist zur sitzenden Aufnahme eines Benutzers vorgesehen. Die Aussparung 304 ist dabei entsprechend einem Rückenbereich des Benutzers angeordnet. Andere Beispiele von Benutzeraufnahmen der vorgestellten Art sind davon abweichend oder zusätzlich zur stehenden und/oder liegenden Aufnahme eines Benutzers vorgesehen. Zudem ist in anderen Beispielen von Benutzeraufnahmen der vorgestellten Art wenigstens eine Aussparung entsprechend einem anderen Körperbereich als einem Rückenbereich des Benutzers angeordnet.

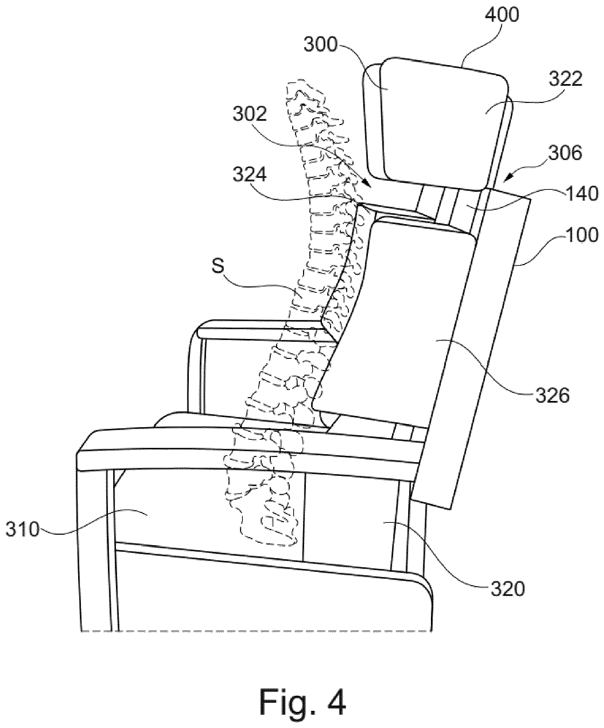

Fig. 4 zeigt schematisch und exemplarisch ein Wärmebehandlungssystem 400. Das Wärmebehandlungssystem 400 umfasst eine Benutzeraufnahme 300 wie im Zusammenhang mit Fig. 3A und 3B beschrieben und eine Infrarotstrahler-Anordnung 100 wie im Zusammenhang mit Fig. 1 und 2 beschrieben. Gleiche Bezugszeichen wie in Fig. 1 bis 3B bezeichnen darin gleiche Merkmale.

Die Infrarotstrahler-Anordnung 100 ist in einem von der Anliegefläche 302 abgewandten Bereich 306 der Benutzeraufnahme 300 an einer Rückseite der Rückenlehne 312 angeordnet. Die Infrarotstrahler-Anordnung 100 ist dabei derart angeordnet, dass die Bestrahlungsrichtung der Infrarotstrahler-Anordnung 100 durch die Aussparung 304 der Benutzeraufnahme 300 in Richtung auf den Benutzer gerichtet ist.

Aus der Position der Infrarotstrahler-Anordnung 100 und der geneigten Rückenlehne 312 ist außerdem erkennbar, dass die Infrarotstrahler-Anordnung 100 dabei Schweißabsonderungen des Benutzers verstärkt ausgesetzt ist. Die Ausrichtung der Infrarotstrahler-Anordnung 100 bewirkt dabei ein Abfließen von auftreffendem Schweiß in Richtung auf den Schweißauffang 130 im unteren Bereich des Gestells 105 der Infrarotstrahler-Anordnung 100.

In Fig. 4 ist exemplarisch die Wirbelsäule S eines Benutzers dargestellt. Dabei ist erkennbar, dass eine Stützung der Wirbelsäule S sowohl durch die Lendenstützen 318, 320 als auch durch die verstellbaren Anliegeelemente 324, 326 erfolgt. Zudem ist erkennbar, dass ein Abstand zwischen der Infrarotstrahler-Anordnung 100 und einem zu bestrahlenden Hautbereich infolge der Krümmungen der Wirbelsäule S variiert. Wie im Zusammenhang mit Fig. 1 beschrieben, begünstigt die Infrarotstrahler-Anordnung 100 dabei jedoch einen gleichmäßigen Wärmeeintrag in verschiedene Bereiche des Bestrahlungsbereichs.

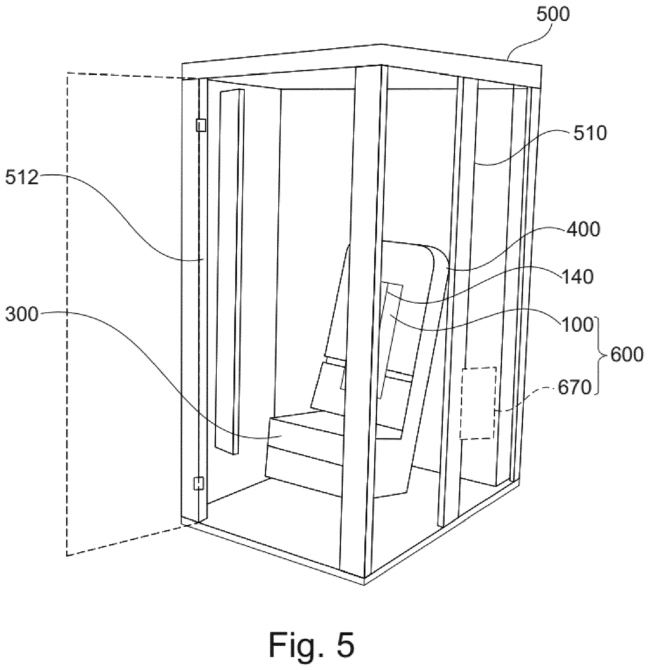

Fig. 5 zeigt schematisch und exemplarisch eine Wärmekabine 500. Die Wärmekabine 500 umfasst eine Kabinenwand 510 und eine Kabinentür 512, die zusammen einen Innenraum der Wärmekabine 500 begehbar umgeben. Die Wärmekabine 500 umfasst außerdem ein Wärmebehandlungssystem 400, das in dem Innenraum der Wärmekabine 500 angeordnet ist.

Bei dem Wärmebehandlungssystem 400 handelt es sich um ein Wärmebehandlungssystem wie voranstehend im Zusammenhang mit Fig. 4 beschrieben. Zur Benutzung des Wärmebehandlungssystems 400 betritt ein Benutzer den Innenraum der Wärmekabine 500 durch die verschließbare Kabinentür 512. Gegenüber einem freistehenden Wärmebehandlungssystem begünstigt die Wärmekabine 500 bei Bedarf eine Durchwärmung des gesamten Körpers des Benutzers.

In einigen Beispielen ist die Wärmekabine 500 als Druckkammer zum Zweck hyperbarer Therapieanwendungen ausgebildet. Die Wärmekabine 500 ist dabei außerdem dazu vorgesehen, Wärmebehandlungen mittels des Wärmebehandlungssystems 400 gleichzeitig mit hyperbaren Therapieanwendungen zu ermöglichen.

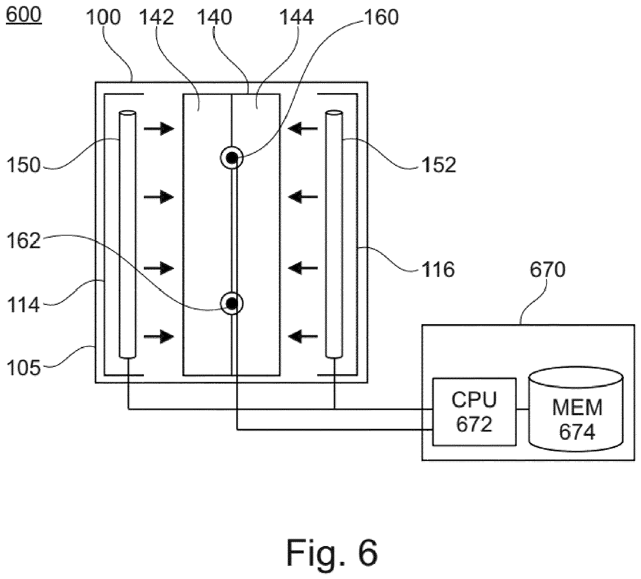

In einigen Beispielen umfasst das Wärmebehandlungssystem 400 zudem eine Steuerungseinrichtung 670, wie in Fig. 5 gestrichelt dargestellt und wie nachstehend im Zusammenhang mit Fig. 6 näher beschrieben. In einigen solcher Beispiele stellen die Steuerungseinrichtung 670 und die Infrarotstrahler-Anordnung 100 zusammenwirkende Teile einer Wärmebehandlungsvorrichtung 600 des Wärmebehandlungssystems 400 dar.

Fig. 6 zeigt schematisch und exemplarisch eine Wärmebehandlungsvorrichtung 600. Die Wärmebehandlungsvorrichtung 600 umfasst eine Infrarotstrahler-Anordnung 100 wie im Zusammenhang mit Fig. 1 und 2 beschrieben und eine Steuerungseinrichtung 670 mit einer Prozessoreinheit 672 und einer Speichervorrichtung 674. Die Steuerungseinrichtung 670 ist mit den Wärmesensoren 160, 162 und mit den Wärmequellen 150, 152, bei denen es sich in diesem Fall um steuerbare Wärmequellen handelt, operativ verbunden.

Die Steuerungseinrichtung 670 ist dazu ausgebildet, eine Heizleistung jeder der Wärmequellen 150, 152 zu steuern. Dazu ist die Steuerungseinrichtung 670 mit den Wärmequellen 150, 152 operativ verbunden. Die Verbindung erfolgt beispielsweise über einen Dimmer (nicht dargestellt), der mittels der Steuerungseinrichtung 670 steuerbar ist. In anderen Beispielen ist die Steuerungseinrichtung 670 dazu ausgebildet, an jede der Wärmequellen 150, 152 einen gemäß einer vorgesehenen Heizleistung bemessenen Heizstrom auszugeben.

Die Steuerungseinrichtung 670 ist ferner dazu ausgebildet, von jedem der Wärmesensoren 160, 162 Sensorsignale zu empfangen, die auf eine mittels des jeweiligen Wärmesensors 160, 162 erfasste Oberflächentemperatur hinweisen. Ein Sensorsignal von jedwedem der Wärmesensoren 160, 162 weist dabei auf eine Temperatur in dem Erfassungsbereich des jeweiligen Wärmesensors 160, 162 hin.

Die Steuerungseinrichtung 670 ist dazu ausgebildet, eine Heizleistung jeder der Wärmequellen 150, 152 wenigstens teilweise auf der Grundlage einer mittels der Wärmesensoren 160, 162 erfassten Oberflächentemperatur in einem Hautbereich des Benutzers, der sich im Bestrahlungsbereich der Wärmebehandlungsvorrichtung 600 befindet, zu steuern, insbesondere temperaturabhängig zu regeln. Dafür ist die Prozessoreinheit 672 dazu programmiert, ein oder mehrere Sensorsignale, die mittels eines oder mehrerer der Wärmesensoren 160, 162 erzeugt und an die Steuerungseinrichtung 670 ausgegeben und von der Steuerungseinrichtung 670 empfangen werden, mit wenigstens einer Bezugstemperatur zu vergleichen und in Abhängigkeit von dem Vergleichsergebnis die Heizleistung wenigstens einer der Wärmequellen 150, 152 zu verändern. Ein Verändern der Heizleistung kann dabei einem Verringern oder einem Erhöhen der Heizleistung entsprechen.

In einigen Beispielen der Wärmebehandlungsvorrichtung 600 ist eine Funktionalität der Prozessoreinheit 672 durch Programmcode bestimmt, der mittels der Speichervorrichtung 674 gespeichert ist und von der Prozessoreinheit 672 ausgeführt wird. In einigen Beispielen erfolgt zudem eine Heizleistungsregelung mittels der Steuerungseinrichtung 670 durch Vergleichen einer erfassten Temperatur mit einer oder mehreren Bezugstemperaturen, die mittels der Speichervorrichtung 674 gespeichert sind.

### Bezugszeichenliste

- 100: Infrarotstrahler-Anordnung
- 105: Gestell
- 110: Boden
- 112: Öffnung
- 114, 116: Seitengehäuse
- 118: Befestigungsmittel
- 120, 122: Wärmesensoraufnahme
- 124, 126: Schweißabtropfkontur
- 128: Schutzgitter
- 130: Schweißauffang
- 132: Schweißauslass
- 140: Reflektor
- 142, 144: Reflektionsfläche
- 146, 148: Reflektoraussparung
- 150, 152: Wärmequelle
- 300: Benutzeraufnahme
- 302: Anliegefläche
- 304: Aussparung
- 306: abgewandter Bereich
- 310: Sitzfläche
- 312: Rückenlehne
- 314, 316: Armlehne
- 318, 320: Lendenstütze
- 322: Nackenstütze
- 324, 326: verstellbares Anliegeelement
- 328, 330: Blendenelement
- 400: Wärmebehandlungssystem
- 500: Wärmekabine
- 510: Kabinenwand
- 512: Kabinentür
- 600: Wärmebehandlungsvorrichtung
- 670: Steuerungseinrichtung
- 672: Prozessoreinheit
- 674: Speichervorrichtung
- B: Bestrahlungsrichtung
- MR: Mittenbereich
- S: Wirbelsäule

## Patentansprüche

1. Benutzeraufnahme (300) für ein Wärmebehandlungssystem (400), die so ausgebildet ist, dass eine Infrarotstrahler-Anordnung (100) des Wärmebehandlungssystems (400) an der Benutzeraufnahme (300) anbringbar ist, wobei die Benutzeraufnahme (300) wenigstens eine Anliegefläche (302) umfasst, die dem Benutzer zugewandt ist und die eine in ihrer Größe und/oder Form verstellbare Aussparung (304) aufweist, die mit einem zur Wärmebehandlung vorgesehenen Hautbereich des Benutzers in einer Bestrahlungsrichtung wenigstens teilweise überlappt, wobei die Infrarotstrahler-Anordnung (100) in einem von der Anliegefläche (302) abgewandten Bereich (306) der Benutzeraufnahme (300) anordenbar ist derart, dass Infrarotstrahlung, die mittels der wenigstens einen Wärmequelle (150, 152) erzeugt ist, durch die in ihrer Größe und/oder Form verstellbare Aussparung (304) in Richtung auf den Benutzer tritt.

2. Benutzeraufnahme nach einem der vorhergehenden Ansprüche, ferner umfassend wenigstens ein Blendenelement (328, 330), dessen Position in Bezug auf die Infrarotstrahler-Anordnung (100) verstellbar ist, um einen Bestrahlungsbereich der Infrarotstrahler-Anordnung (100) verstellbar zu begrenzen.

3. Benutzeraufnahme nach einem der vorhergehenden Ansprüche, wobei die Benutzeraufnahme (300) wenigstens ein verstellbares Anliegeelement (324, 326) umfasst, das die Aussparung (304) in wenigstens einer Richtung verstellbar begrenzt.

4. Benutzeraufnahme nach Anspruch 3, wobei das wenigstens eine verstellbare Anliegeelement (324, 326) gemäß einer Körpergröße des Benutzers und/oder gemäß einer Körperregion des zur Wärmebehandlung vorgesehenen Hautbereichs des Benutzers verstellbar ist.

5. Benutzeraufnahme nach Anspruch 3 oder 4 in Kombination mit Anspruch 2, wobei das wenigstens eine Blendenelement (328, 330) an einer von der Anliegefläche (302) abgewandten Seite des wenigstens einen verstellbaren Anliegeelements (324, 326) angeordnet ist, und die Position des Blendenelements (328, 330) in Bezug auf die Infrarotstrahler-Anordnung (100) verstellbar ist durch Verstellen des verstellbaren Anliegeelements (324, 326).

6. Benutzeraufnahme nach Anspruch 5, wobei das wenigstens eine verstellbare Anliegeelement (324, 326) mit dem wenigstens einen Blendenelement (328, 330) verstellbar ist, um den Bestrahlungsbereich der Infrarotstrahler-Anordnung (100) gemäß einer Größe des zur Wärmebehandlung vorgesehenen Hautbereichs des Benutzers verstellbar zu begrenzen.

7. Benutzeraufnahme nach einem der vorhergehenden Ansprüche, wobei die Benutzeraufnahme (300) eine Stützstruktur in Form eines Sitzes, einer Liege und/oder einer Lehne umfasst und zur sitzenden, liegenden und/oder stehenden Aufnahme des Benutzers vorgesehen ist.

8. Benutzeraufnahme nach Anspruch 7, wobei die Anliegefläche (302) einer dem Benutzer zugewandten Seite der Stützstruktur entspricht, und die Infrarotstrahler-Anordnung (100) im Bereich einer von der Anliegefläche (302) abgewandten Seite der Stützstruktur anbringbar ist.

9. Benutzeraufnahme nach Anspruch 7 oder Anspruch 8, wobei die Aussparung (304) sich in einem Bereich der Stützstruktur erstreckt, der einem Rückenbereich des Benutzers entspricht.

10. Benutzeraufnahme nach Anspruch 9 in Kombination mit einem der Ansprüche 2 bis 6, wobei das wenigstens eine Blendenelement (328, 330) und/oder das wenigstens eine verstellbare Anliegeelement (324, 326) in wenigstens einer aus einer vertikalen und einer lateralen Richtung verstellbar ist, um eine Größe des zur Bestrahlung vorgesehenen Hautbereichs im Rückenbereich des Benutzers einzustellen und/oder um die Stützstruktur einer Körpergröße des Benutzers anzupassen.

11. Benutzeraufnahme nach Anspruch 10, umfassend wenigstens zwei Blendenelemente (328, 330) und/oder wenigstens zwei verstellbare Anliegeelemente (324, 326), die lateral zu verschiedenen Seiten eines Mittenbereichs der Aussparung (304) angeordnet sind.

12. Wärmebehandlungssystem (400) umfassend:
eine Benutzeraufnahme (300) nach einem der vorhergehenden Ansprüche, und
eine Infrarotstrahler-Anordnung (100), die in Bezug auf die Anliegefläche (302) der Benutzeraufnahme (300) derart angeordnet ist, dass Infrarotstrahlung, die mittels der Infrarotstrahler-Anordnung (100) erzeugt ist, durch die Aussparung (304) in Richtung auf den Benutzer tritt.

13. Wärmebehandlungssystem nach Anspruch 12, wobei die Infrarotstrahler-Anordnung (100) umfasst:
wenigstens eine Wärmequelle (150, 152),
wenigstens einen Reflektor (140, 142, 144), der dazu ausgebildet ist, Infrarotstrahlung, die mittels der wenigstens einen Wärmequelle (150, 152) erzeugt ist, in Richtung auf den Benutzer des Wärmebehandlungssystems (400) umzulenken, und
wenigstens ein Gestell (105), an dem die wenigstens eine Wärmequelle (150, 152) und der wenigstens eine Reflektor (140, 142, 144) angebracht sind,
wobei während einer vorgesehenen Verwendung des Wärmebehandlungssystems (400) der wenigstens eine Reflektor (140, 142, 144) mechanischer und/oder chemischer Wechselwirkung mit einer Umgebung des Wärmebehandlungssystems (400) ausgesetzt ist, und der wenigstens eine Reflektor (140, 142, 144) reversibel lösbar an dem wenigstens einen Gestell (105) angebracht ist.

14. Wärmebehandlungssystem nach Anspruch 13, wobei die wenigstens eine Wärmequelle (150, 152) eine steuerbare Wärmequelle (150, 152) ist, und wobei das Wärmebehandlungssystem (400) ferner umfasst:
wenigstens einen Wärmesensor (160, 162), der in der Infrarotstrahler-Anordnung (100) aufgenommen ist und der dazu ausgebildet ist, während der vorgesehenen Verwendung des Wärmebehandlungssystems (400) eine Temperatur in dem Hautbereich des Benutzers zu erfassen und ein Sensorsignal auszugeben, das auf die erfasste Temperatur hinweist, und
eine Steuerungseinrichtung (670), die dazu ausgebildet ist, das Sensorsignal zu empfangen und wenigstens teilweise auf der Grundlage des empfangenen Sensorsignals die wenigstens eine Wärmequelle (150, 152) in Bezug auf eine Heizleistung zu steuern,
wobei die wenigstens eine Wärmequelle (150, 152), der wenigstens eine Reflektor (140, 142, 144), der wenigstens eine Wärmesensor (160, 162) und die Steuerungseinrichtung (670) Bestandteile einer Wärmebehandlungsvorrichtung (600) des Wärmebehandlungssystems (400) sind.

15. Wärmekabine (500) umfassend ein Wärmebehandlungssystem (400) nach einem der Ansprüche 12 bis 14.
